# EUROPEAN PATENT APPLICATION

(11) **EP 0 987 023 A1**
(43) Date of publication of application: **22.03.2000**
(21) Application number: 98610026.1
(22) Date of filing: 17.08.1998
(51) Int. Cl.: A61K 31/315, A61K 31/19

(54) **Transition metal complexes of non steroidal anti-inflammatory drugs**

(71) Applicant: PANACEA BIOTEC LIMITED, New Dehli 110001 (IN)
(72) Inventor: Amarjit, Singh, New Dehli-110001 (IN); Rajesh Jain, New Dehli-110001 (IN); Anil,KumarSingla c/o Ms.Chagumal Santram, Punjab (IN)
(74) Representative: Christiansen, Ejvind

(57) **Abstract**

A COX ₂ selective pharmaceutical composition is disclosed. The composition has a potency ratio less than 1 and comprises a complex of Naproxen and/ or one or more salts and/or adducts of Naproxen or mixtures thereof and Zinc in one or more salt forms or mixtures thereof and represented by the formula -

( Drug)₂ Zn.nH₂O.

The drug in the above formula is Naproxen in a suitable pharmaceutical base/carrier or diluent.

## Description

### INTRODUCTION

The present invention relates to a novel pharmaceutical composition comprising Naproxen and a transition metal. The Novel drug metal complex is characterised in having increased efficacy and manifolds better tolerability than the parent drug and standard NSAIDS. More particularly the invention relates to pharmaceutical complex of Naproxen and Zinc, obtained by reacting Naproxen and (or) one or more salts of Naproxen with Zinc in one or more salt form(s).

### BACKGROUND OF THE INVENTION

Naproxen (US patents 3,904,682 & 4,009, 197) is (+) -2-(6-Methoxy-2-haphthyl) propionic acid [I.T. Harrison et al J. Med. Chem., 13 203 (1970)]. It has analgesic, antiinflammatory and antipyretic properties. It is an inhibitor of Cyclo-oxygenase [(Roszkowski et al Pharmacol. Exp. Ther 179, 114 (1971)],[Tomlinson et al Biochem Biophys REs commun 46 552 (1972)]. Both Naproxen and Naproxen Sodium are used in muscoskeletal and joint disorders such as ankylosing spondylitis, osteoarthritis and rheumotoid arthritis, in mild to moderate pain such as dysmenorrhoea, migraine and some musculoskeletal disorders, and in acute gout [R.N. Brogden et al Drugs, 18, 241-277 (1979)]. In some of the conditions the drug has to be administered for long periods of time. Among the other adverse-effects, Gastro-intestinal adverse effects are among the most frequently reported during short and long term treatment with Naproxen. Hence development of the pharmaceutical forms which reduce the adverse effects, in particular the Gastro-intestinal adverse effects is most desirable.

There are several references of different types of approaches towards improvement of galenic dosage forms of Naproxen such as programmed release (US Patent Nos. 5480650, 4888178), Diffusion-osmotic controlled drug-release (US patent nos. 5543155, 4571333), Liquid-suspension controlled release (US Patent no. 5527545) Systems for improved bio-availability or increased patient compliance e.g Naproxen micro capsules (W.O. Patent 9505166) and Cyclodextrin inclusion compounds (W.O patent. 9504528) have also been reported. Soluble salts of Naproxen have been prepared with N-(2-hydroxy ethyl) pyrrolodone (U.S. patent no. 5206262).

Drug ligand-metal complexes of several therapeutic compounds have been reported. In several cases the ligand-metal complexes have better therapeutic and/or pharmaceutical properties than the parent ligand. The prior art regarding this aspect for NSAIDS is descussed below.

An inclusion complex of a NSAID or a pharmaceutically acceptable Salt thereof and a Cyclodextrin, and a physiologically acceptable alkali agent selected from the group consisting of alkali and alkaline earth metal carbonates, bicarbonates, phosphate and hydroxides, and water-soluble amines have been described in WO patent no. 9614839. The alkali agent has been shown to be capable of forming an alkaline diffusion layer around the composition in the gastro-intestinal tract.

Chen et al (J. Inorg. Biochem. 1992 Aug; 47 (2); 81-7) have described synthesis, characterisation, and anti-inflammatory activity of Naproxen complexes with rare earth (III). Various analyses have indicated presence of 1:3 (metal: ligand) stochiometry and the carboxylate group of Naproxen has been suggested as a bridging ligand to co-ordinate to RE (III) ions. Two pharmacological models were chosen to examine the antiinflammatory activity of Nd(III) complex, which ascertained enhanced anti-inflammatory activity relative to the ligand.

It has been known now for 60 years that Zinc is essential for animal (including human) life. Approximately 300 enzymes require Zinc for their activities and it has a role in DNA Synthesis, Cell division and protein synthesis (Peasad A, Nutrition 1995, 11, 93-9). The inventors have looked into the possibility of making Zinc complexes of NSAIDs, particularly of Naproxen which have not been reported so far

Navarro et al (Prostaglandins Leukot. Essent. Fatty Acids, 1994 Jun; 50 (6); 305-10) studied the effect of pre-treatment with Zinc acexarnate on Gastrotoxic activities of different NSAIDs (diclofenac, indomethacin, Ketoprofen, naproxen and piroxicam). Zinc acexamate pre-treatment significantly decreased the overall Severity of lessions induced by NSAIDS. The experiments Corroborate the hypothesis that the preservation of the capability to synthesise endogenous prostaglandins is of critical importance in the maintenance of gastric mucosal integrity. The gastro-protective action observed with Zinc acexamate involves alternative mechanisms other than modification of PGE levels.

US Patent no. 5466824 describes a process for the preparation of a complex of indomethacin and a divalent metal comprising forming a solution by dissolving indomethacin and a salt of said divalent metal in a tertiary amide or cyclic teritory amide, adding a C₁₄ alkanol or C₃₋₆ Ketone to the solution to precipitate the complex, and separating the precipitated complex from the solution. This art also provides a method for the treatment of inflammation and/or pain in a mammal requiring such treatment, comprising administering to said mammal an anti-inflammatory or analgesically effective amount of a complex of indomethacin and a divalent metal, the complex having the formula (M₂) (Indomethacin₄) (Sₙ) wherein M is the divalent metal, S is a molecule of tertiary amide or a cyclic tertiary amide, and n is 2 or 3, or of a pharmaceutical composition comprising said complex together with a pharmaceutically acceptable carrier, diluent and/or excipient. This US patent further disclosures a complex of indomethacin and a divalent metal, the complex having the formula [M₂] [indomethacin₄] [Sₙ], wherein M, S, and n are defined above, and a pharmaceutical composition comprising this complex together with a pharmaceutically acceptable carrier, diluent and/or excipient.

The zinc-aspirin complex has been reported by A.K. Singla et. al. (International Journal of Pharmaceutics, 108 (1994), 173-185). The complex was reported to have better efficacy and tolerability profile. Further Zinc-indomethacin complex has been reported (A.K. Singla et. al. International Journal of Pharmaceutics, 120 (1995) 145-155. The zinc-indomethacin complex was more potent than the parent drug and also had a better tolerability profile with respect to ulcerogenic properties.

U.S. Patent No. 5466824 describes a complex formation of indomethacin with a divalent metal and a tertiary amide or a cyclic tertiary amide while that to C₁₄ alkanol or C₃₋₆ Ketone. Inclusion of alien molecules in a complex changes the total course of drug development process and substantial data in order to prove safety and efficacy of a new complex is essential. The process of preparation itself in U.S. Patent No. 5466824 is very cumbersome, time comsuming, costly and the presence of new entities besides the drug and the divalent metal may take considerable time and pre-clinical and clinical tests before the drug can be put to usefulness.

One of the inventors of the group of inventors of the present application has reported the formation of zinc-NSAID complexes. The zinc-indomethacin has been shown to be better than the parent molecule. Similarly, the aspirin-zinc complex has been shown to be better than the parent molecule. However both these NSAIDs drugs have their own disadvantages in long term use. Indomethacin has low therapeutic index while aspirin has to be used in very high dosage to produce anaphylactic reactions. Although the zinc salts complexes are better, they may not be useful in therapeutics.

Besides indomethacin is not commonly used for therapy as an analgesic or antipyretic because of high incidence and severity of side effects associated with long term administration. It is reserved for special use in acute gout and severe ankylosing spondylitis and osteoarthritis. This is evident from ED₅₀, LD₅₀, and therapeutic index, values of NSAIDs as collected herein below.

| **S.No.** | **Drug** | **ED**_{**50**}**(mg/kg)** | **LD**_{**50**}**(mg/kg)** | **Therapeutic Index** |
|---|---|---|---|---|
| 1. | Nimesulide | 1.25 | 324 | 260 |
| 2. | Naproxen | 2.10 | 395 | 190 |
| 3. | Ibuprofen | 13.5 | 923 | 68 |
| 4. | Diflumidone | 38.0 | 750 | 20 |
| 5. | Flufenamic acid | 14.7 | 249 | 17 |
| 6. | Phenylbutazone | 20.5 | 406 | 14 |
| 7. | Acetylsalicylic acid | 135.0 | 1520 | 14 |
| 8. | Indomethacin | 2.95 | 210 | 7 |

Early approaches for improvement in efficacy and side effect profile were targetted with respect to drugs with low therapeutic index and poor safety margin.

NSAIDs can be ranked by concentration (µg/ml) of drug necessary to achieve 50% inhibition (IC₅₀) of COX₂ divided by IC₅₀ for COX₁, and the values below 1 indicate selectivity for COX₂. The similarities in rates of g.i. complications were found to be as striking as the difference in ratio of COX₁ and COX₂ inihitory action as illustrated below.

The data as per the reference "Drugs of Today Vol. 32 Suppl. D1996 Page 5" as illustrated herein below shows that only four compound out of which Naproxen and Diclofenac are examples are selective for COX₂ potency ratio < 1. Among the many available NSAIDs, indomethacin and aspirin are COX₁ selective with potency ratio upto 100.

| Drug | Cyclooxygenase 2/Cyclooxygenase 1 ratio |
|---|---|
| Piroxicam | 250 |
| Tolmetin | 175 |
| Acetylsalicylic acid | 166 |
| Sulindac | 100 |
| Indomethacin | 60 |
| Tolfenamic acid | 16.7 |
| Ibuprofen | 15 |
| Paracetamol | 7.4 |
| Sodium salicylate | 2.8 |
| Flurbiprofen | 1.3 |
| Carprofen | 1 |
| Meloxicam | 0.8 |
| Diclofenac | 0.7 |
| Naproxen | 0.6 |
| Nimesulide | 0.1 |

On the other hand while Naproxen has a comparative COX₂/COX₁ ratio (0.6) which is much lesser in comparision to other NSAIDs such as aspirin (166) and indomethacin (60).

After careful and planned experimentation and with a quest to a improve upon the drug candidates with relatively high therapeutic index and relatively high safety margin, the inventors of the present invention conceived, planned and executed a research project to evaluate the zinc complexation on safety and efficacy of such drug canditates by taking examples of Naproxen and diclofenac.

The compositions of the present invention are therapeutically useful efficacious and safe, simple to manufacture and the process of manufacture is not time consuming. Besides the entire process and the drug manufactured thereby is not expensive.

### SUMMARY OF THE INVENTION

The present invention discloses a novel pharmaceutical COX₂ selective composition having potency ratio less than 1 of Naproxen and/or one or more salts and/or adducts of Naproxen and zinc in one or more salt forms.

### DETAILED DESCRIPTION OF THE INVENTION

The inventors have synthesised Zinc-diclofenac complex. This complex was found to have a more potent anti-inflammatory activity than diclofenac but it showed more toxicity in experimental animals.

In accordance with the present invention the pharmaceutical composition comprising Naproxen and zinc can be represented by the general formula (Drug)₂Zn.nH₂O. In other words two molecules of the drug are reacted with one molecule of zinc in the presence of water.

The salts and adducts of Naproxen and Diclofenac are selcted from the group optically pure Naproxen, Naproxen-Lysine salt, Naproxen-Cyclodextrin Adduct, Diclofenac Potassium, Diclofenac Diethylammonium, Diclofenac Epolamine and Diclofenac Tromethamine.

The salts of zinc are selected from zinc carbonate, zinc acetate, zinc sulphate, zinc chloride and zinc glucomate.

In accordance with another aspect of the present invention, is disclosed the synthesis of Naproxen-Zinc complex. After careful and planned experimentation on studies of pharmacological properties of the metal-ligands prepared it has been surprisingly found that Naproxen-Zinc complex have Synergistically higher anti-inflammatory and analgesic activities when compared to the parent drugs, Zinc alone and physical mixture of the drug and Zinc. There is a trend that as anti-inflammatory and analgesic properties of a NSAID compound increase, the side effects particularly the gastric-irritation also increases. The phenomena is attributed to inhibition of endogenous PEG₂ in gastric mucosa. Moreover it has been further surprisingly found that Naproxen-Zinc complex has least gastrotoxic side effects measured by ulcer index although it has a very high anti-inflammatory and analgesic activity.

In another embodiment of the present invention the composition is disclosed where the Naproxen is converted into a therapeutically safe and efficacious form i.e. Naproxen-zinc just before administration through oral or other construable routes. Such compositions are very simple to prepare, cost effective and useful. Such compositions have two parts. In one part Naproxen is present in a suitable pharmaceutical carrier and the other part contains a titrated reactive form of zinc with suitable pharmaceutical carrier. In such a composition both the parts are separately reacted in a suitable amount of medium or are present in a admixture form which is reacted just prior to administration.

The Naproxen-zinc complex was prepared by reacting Zinc Carbonate and Naproxen Sodium in primarily aqueous solvent, which may contain one or more polar organic solvent, in a molar ratio such that two moles of Naproxen react with one mole of Zinc. The complex thus formed was separated and dried.

The dried compound was subjected to elemental analysis, Infra-Red Spectroscopy Nuclear Magnetic Resosance spectroscopy and Differential Scanning Calorimetory (DSC). The formation of Naproxen Zinc complex was proved by comparing IR scans and DSC graphs of the complex with individual compound separately Examination of the IR-spectra of Naproxen and the synthesised Zinc-Naproxen complex revealed a definite shift in absorption for the carboxyl group and the disappearance of carboxyl O-H stretching and bending. The shift occurred in the direction of longer wavelength, indi cating that the carboxyl group of Naproxen is strongly involved in complexation with Zinc. Comparison of the H-NMR spectra of the two compounds did not show much difference as in the case of Naproxen due to exchange broadening involving the water in the solvent. The formation of the Zinc complex was further confirmed by C¹³-NMR spectra which revealed a strong shift in the absorption of -COOH and slight shifts in the case of other carbon atoms. DSC curves for Naproxen, the physical mixture of Naproxen and Zinc sulphate, and Zinc-Naproxen complex, showed that the endothermic peak of Naproxen disappeard completely in the Zinc complex with the appearance of two new endothermic peaks. The first peak is due to loss of water of crystallisation and the second sharper peak is due to melting with decomposition of the Zinc complex.

Diclofenac, Naproxen, Diclofenac-Zinc and Naproxen Zinc along with normal saline (as control) and Indomethacin (as standard Anti-inflammatory drug) were subjected to the pharmacological screening tests.

The anti-inflammatory of the compounds was tested by Carageenan - induced rat paw edema and ulcerogenic effect was evaluated by finding ulcer index.

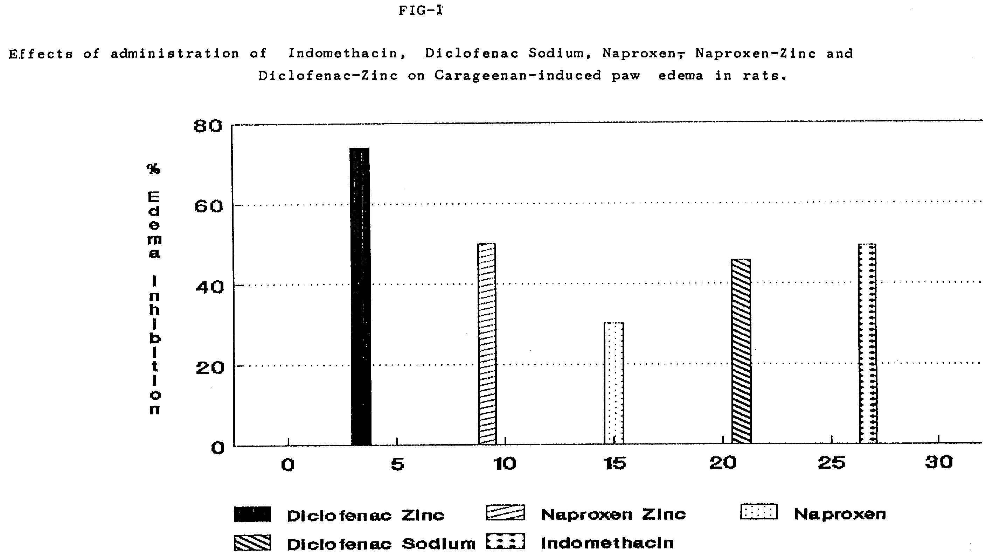

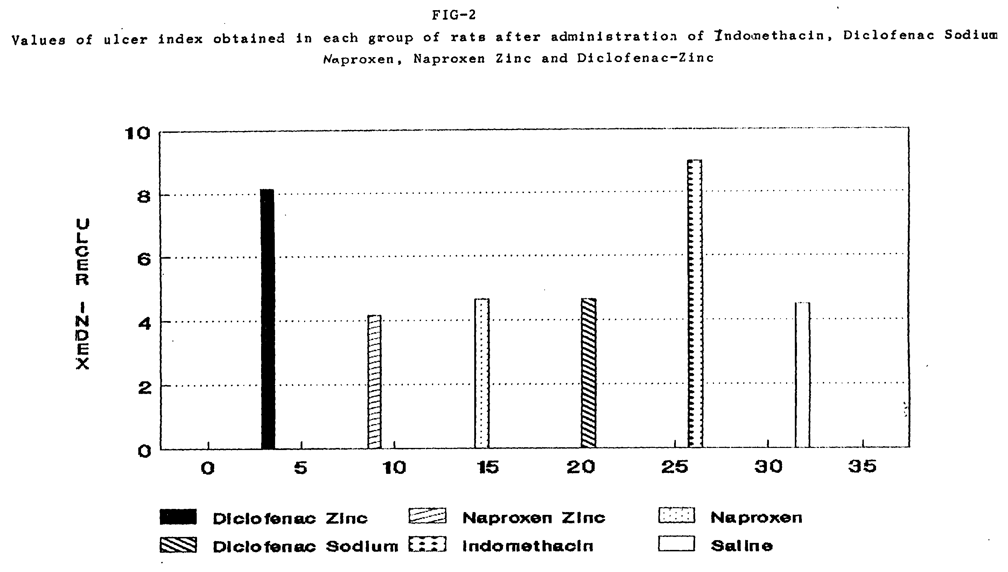

The findings of the study are summarised in table 1 & 2 and depicted in fig. 1 & 2.

The invention provides Naproxen-Zinc complex and a process for manufacture thereof not reported earlier and the initial pharmacological testing indicates a strong possibilities that this complex may be developed as a therapeutically useful compound with reduced dry dose and better tolerability w.r.t gastric irritation.

### PREPARATION OF ZINC-NAPROXEN COMPLEX

Naproxen (69.1 g, 0.3 ml) was dissolved in a solution of sodium bicarbonate (25.2 g, 0.3 ml) in water (500 ml) and filtered (pH 8.14). To this solubility was addeed slowly and with constant stirring a solution of zinc acetate dihydrate (35 g, 0.15 ml) in water (180 ml). Immediate precipitation occurred and the precipitates were filtered, washed with a minimum quantity of absolute alcohol and then with cold water, and dried under vaccum to a constant weight to give zinc-naproxen complex (yield, 69.75g, 83.1%); m.p. 222 - 224°C.

### CHARACTERISATION OF ZINC-NAPROXEN COMPLEX

Comparison of the IR spectra of naproxen and its zinc complex showed the disappearance of Carboxyl OH stretching (3166cm⁻¹) in the IR spectrum of the complex occurence of strong shift in the absorption due to C = O group stretching (1727 to 1689 cm⁻¹) and the shift in -CH-COOH stretch from 1175 to 160 cm⁻¹ also indicating the possibility of host guest interchanging at this site. The displacement occured in the directions of longer wavelength indicating that the carboxyl group of naproxen is strongly involved in complexation with zinc. Donation of electron to metal produces lower excitation states and therefore shifts to longer wavelengths (William et al., 1976)

The inventors effected the experiments to test the anti-inflammatory activity and toxicity by evaluating the ulcer index employing Naproxen-Zinc complex and Diclofenac-Zinc complex synthesised by them. It has been suprisingly found by the inventors that the anti-inflammatory activity of the Naproxen-Zinc and Diclofenac-Zinc complex were much more than the parent drug. However, Diclofenac Zinc complex suffered from the char acteristic of being toxic, the toxicity being proportional to the increase in its anti-inflam matory activity.

The invention will now be described with reference to the following examples which are by way of illustration:

### Example 1 : Tablet dosage form

| | |
|---|---|
| Naproxen Zinc equivalent to Naproxen 125 mg | 152.25 mg |
| Lactose | 229.15 mg |
| Starch | 30.00 mg |
| PVP | 10.00 mg |
| Micro Crystalline Cellulose | 15.00 mg |
| Purified Talc | 0.90 mg |
| Magnesium Stearate | 12.00 mg |
| | 300.00 mg |

### Example 2 : Tablet dosage form

| | |
|---|---|
| Naproxen Zinc equivalent to Naproxen 250 mg | 304.50 mg |
| Lactose | 110.50 mg |
| HPMC | 30.00 mg |
| Micro Crystalline Cellulose | 45.00 mg |
| Purified Talc | 10.00 mg |
| | 500.00 mg |

### Example 3 : Tablet dosage form

| | |
|---|---|
| Naproxen Zinc equivalent to Naproxen 375 mg | 456.75 mg |
| Micro Crystalline Cellulose | 148.25 mg |
| HPMC | 30.00 mg |
| Magnesium Stearate | 15.00 mg |
| | 650.00 mg |

### Example 4 : Tablet dosage form

| | |
|---|---|
| Naproxen Zinc equivalent to Naproxen 500 mg | 609.00 mg |
| Lactose (Directly compressible) | 141.00 mg |
| | 750.00 mg |

### Example 5 : Tablet dosage form

| | |
|---|---|
| Diclofenac Zinc equivalent to Diclofenac 50 mg | 56.85 mg |
| Lactose | 90.00 mg |
| Micro Crystalline Cellulose | 30.00 mg |
| Polyvinylpyrrolidone | 7.15 mg |
| Sodium Starch Glycollate | 16.60 mg |
| | 200.00 mg |

### Example 6 : Capsule dosage form

| | |
|---|---|
| Naproxen Zinc equivalent to Naproxen 250 mg | 304.50 mg |
| Micro Crystalline Cellulose | 133.00 mg |
| Magnesium Stearate | 7.00 mg |
| Sodium Lauryl Sulphate | 1.5 mg |
| | 500.00 mg |

Filled in size '0' hard gelatin capsules shells.

### Example 7 : Suspension dosage form

| | |
|---|---|
| Naproxen Zinc equivalent to Naproxen 125 mg | 152.25 mg |
| Cane Sugar | 1000.00 mg |
| Glycerine | 200.00 mg |
| Sodium Saccharin | 4.00 mg |
| Dr. Sodium EDTA | 8.00 mg |
| Xanthan GUM | 100.00 mg |
| Pineapple Flavour | 4.00 mg |
| Purified Water q.s. to | 5.00 ml |

### Example 8 : Enteric Coating Tablet

| | |
|---|---|
| Diclofenac Zinc equivalent to Diclofenac 25 mg | 28.42 mg |
| Lactose | 100.00 mg |
| Micro Crystalline Cellulose | 71.58 mg |
| HPMC phthalate | 36.00 mg |
| Isopropyl Alcohol* | |
| | 236.00 mg |

| | |
|---|---|
| * Lost in process | |

### Example 9 : Chewable Tablet Dosage form

| | |
|---|---|
| Naproxen Zinc equivalent to Naproxen 375 mg | 456.75 mg |
| Sugar Granular | 365.25 mg |
| Mannitol | 100.00 mg |
| Starch | 60.00 mg |
| Mixed Fruit Flavour | 10.00 mg |
| Aspartame | 2.00 mg |
| Magnesium Stearate | 6.00 mg |
| | 1000.00 mg |

### Example 10 : Effervscent Tablet

| | |
|---|---|
| Naproxen | 250.00 mg |
| Zinc Carbonate | 80.00 mg |
| Sodium Chloride | 10.00 mg |
| PEG 400 | 10.00 mg |
| | 350.00 mg |

### Example 11 : Effervscent Tablet

| | |
|---|---|
| Naproxen | 500.00 mg |
| Citric Acid | 60.00 mg |
| Zinc Carbonate | 250.00 mg |
| Sodium Chloride | 20.00 mg |
| PEG 400 | 20.00 mg |
| | 850.00 mg |

### Example 12 Capsules dosage form

| | |
|---|---|
| Naproxen-Zinc Cyclodextrin Complex | 375.00 mg |
| Lysine | 115.00 mg |
| Magnesium Stearate | 5.00 mg |
| Sodium Lauryl Sulphate | 5.00 mg |
| | 500.00 mg |

Fill in size '0' hard gelatin capsule shells

### Example 13. Kit

| Granules A | |
|---|---|
| Naproxen | 250.00 mg. |
| Citric Acid | 30.00 mg. |
| PEG 400 | 20.00 mg. |
| Zinc Acetate | 300.0 mg. 130.00 mg. |

| Granules B. | |
|---|---|
| Zinc Acetate | 130.00 mg. |
| Sodium Bicarbonate | 92.00 mg. |
| Sodium Chloride | 28.00 mg. |

Dissolve B in a glass of water and add A. Administer.

### Example 14. Topical formulation

| | |
|---|---|
| Naproxen Zinc | 1% w/v |
| Preservative | 0.005 to 0.5% w/v |
| Buffer/vehicle | 98.5 to 98.995% w/v |

The examples of the formulation should not be construed to limit the scope of the invention. In fact following these examples, any desired pharmaceutical formulation containing Naproxen and zinc can be prepared. The composition of the invention can be in any form commonly employed for administration i.e drink solution, a concentrated drink solution to be diluted before use, solution encapsulated in soft gelatin capsules, solution adsorbed on suitable adsorbents leading to formulations such as tablets, capsules, and granules, the solution freeze dried for oral, topical solution, or Injectable dosage forms or the like.

Another embodiment of this invention is a kit which comprises one or more pharmaceutically acceptable doses of Naproxen and/ or one or more salts and/or adducts of Naproxen or mixtures thereof and one or more acceptable doses of Zinc in one or more salt forms or a mixture thereof.

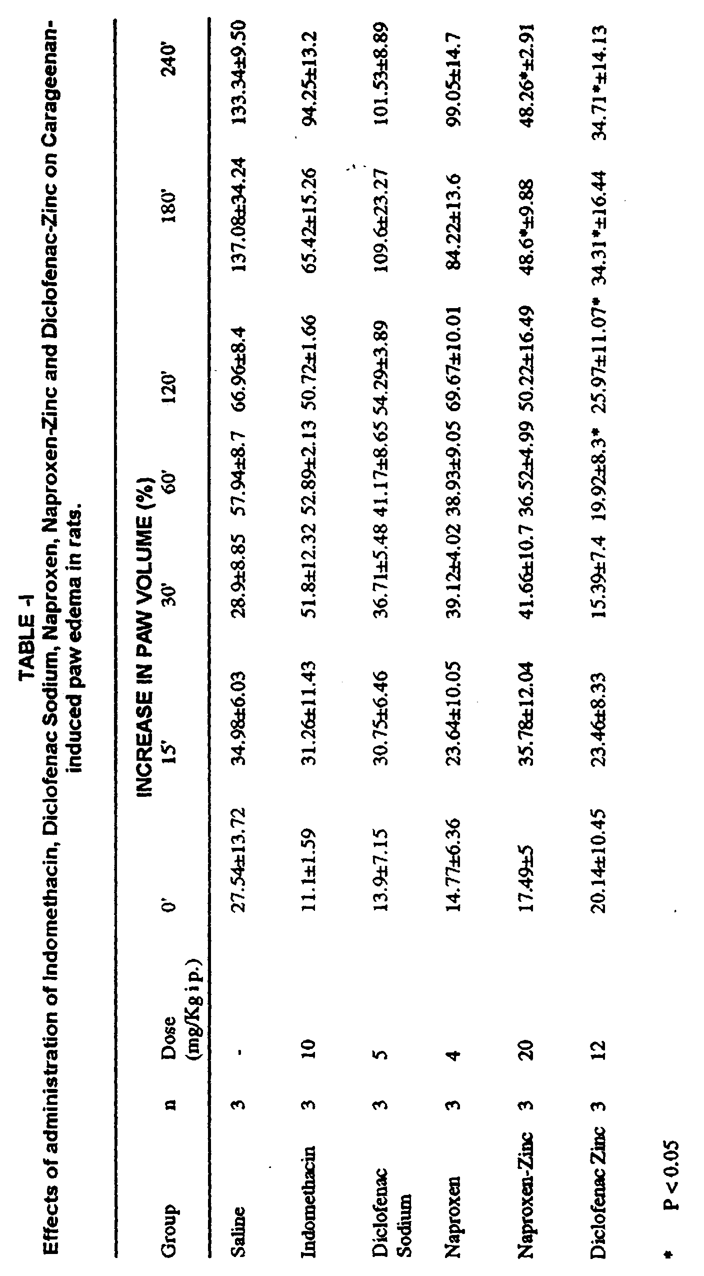

**TABLE 2**

| **Values of ulcer index obtained in each group of rats after administration of Indomethacin, Diclofenac Sodium, Naproxen, Naproxen Zinc and Diclofenac-Zinc** | | | |
|---|---|---|---|
| Group | n | Dose (mg/kg. p.o) | Ulcer Index |
| Saline | 3 | - | 4.5±0.86 |
| Indomethacin | 3 | 10 | 09±2.5 |
| Diclofenac Sodium | 3 | 5 | 4.66±0.88 |
| Naproxen | 3 | 4 | 4.66±1.01 |
| Naproxen-Zinc | 3 | 20 | 4.16±0.44 |
| Diclofenac-Zinc | 3 | 12 | 8.16±0.83* |

| | | | |
|---|---|---|---|
| * p < 0.05 | | | |

## Claims

1. A COX ₂ selective pharmaceutical composition having potency ratio less than 1 comprising a complex of Naproxen and/ or one or more salts and/or adducts of Naproxen or mixtures thereof and Zinc in one or more salt forms or mixtures thereof and represented by the formula -
( Drug)₂ Zn.nH₂O
wherein the drug is Naproxen in a suitable pharmaceutical base/carrier or diluent.

2. A composition as claimed in claim 1 wherein the salts and adducts of Naproxen are selected from the group comprising optically pure Naproxen, Naproxen-Lysine salt and Naproxen Cyclosporin adduct.

3. A composition as claimed in claim 1 wherein the salt forms of Zinc are selected from the group comprising Zinc carbonate, Zinc acetate, Zinc sulphate, Zinc chloride and Zinc glucomate.

4. A composition as claimed in claim 1 which is in the form of a tablet, capsule, suspension, enteric coating tablet, chewable tablet, effervescent tablet and a topical formulation.

5. A kit comprising one or more pharmaceutically acceptable doses of Naproxen and/ or one or more salts and/or adducts of Naproxen or mixtures thereof and one or more acceptable doses of Zinc in one or more salt forms or a mixture thereof.

6. A method of treating an NSAID-indicated condition or symptom with reduced gastrointestinal side effects, said methods comprising: administering an effective amount of a complex of Naproxen, its salt or adduct, or mixtures thereof, and zinc or a salt thereof, said complex represented by the formula-
(Drug)₂ Zn.nH₂O
wherein the drug is Naproxen in a suitable pharmaceutical base/carrier or diluent.

7. A process for the manufacture of a COX₂ selective pharmaceutical composition having potency ratio less than 1 comprising a complex of Naproxen and/ or one or more salts and/or adducts of Naproxen or mixtures thereof and Zinc in one or more salt forms or mixtures thereof and represented by the formula -
( Drug)₂ Zn.nH₂O
wherein the drug is Naproxen in a suitable pharmaceutical base/carrier or diluent which comprises mixing Naproxen and/ or one or more salts and/or adducts of Naproxen or mixtures thereof and Zinc in one or more salt forms or mixtures thereof in the presence of water under conventional conditions of temprature and pressure.

8. A process as claimed in claim 7 wherein the salts and adducts of Naproxen are selected from the group comprising optically pure Naproxen, Naproxen-Lysine salt and Naproxen Cyclosporin adduct.

9. A process as claimed in claim 7 wherein the salt forms of Zinc are selected from the group comprising Zinc carbonate, Zinc acetate, Zinc sulphate, Zinc chloride and Zinc glucomate.

10. A process as claimed in claim 7 wherein Naproxen is dissolved in a solution of Sodium bicarbonate in water and filtered prior to mixing with a mixture of Zinc and salts thereof.

11. A process as claimed in claim 7 wherein Zinc in one or more salt forms or mixtures thereof are mixed with Naproxen and.its salts or adducts or mixtures thereof under constant stirring.
